# EUROPEAN PATENT SPECIFICATION

(11) **EP 1 626 115 B1**
(45) Date of publication and mention of the grant of the patent: **07.05.2014**
(21) Application number: 05380166.8
(22) Date of filing: 26.07.2005
(51) Int. Cl.: D04H 13/00

(54) **Fabric of waterproof breathable microporous membrane**
Tissu et membrane imperméable microporeuse apte à la respiration
Gewebe aus einer wasserbeständigen atmungsfähigen mikroporösen Membran

(30) Priority: 30.07.2004 ES 200401889
(43) Date of publication of application: 15.02.2006
(73) Proprietor: Projectes Baspa, S.L., 08750 Molins de rei, Barcelona (ES)
(72) Inventor: Bassons Boncompte, Jaime, 08750 Molins de rei (Barcelona) (ES)
(74) Representative: Durán Moya, Luis-Alfonso

(56) References cited:
- EP-A- 0 895 766
- EP-A- 0 906 824
- EP-A- 1 040 806
- WO-A-03/047489

## Description

The present invention relates to a fabric, to a process for obtaining it and to undergarments obtained from it.

In particular, it relates to a fabric which comprises at least two layers, one of which is waterproof.

Fabrics of this type are used in all kinds of applications in which a fabric which prevents the passage of liquids is required.

Examples of this type of application include tablecloths, mattress covers, sofa protectors, hospital beds or any type of garment in which the user produces secretions derived from menstruation, incontinence, lactation, post-operative processes, etc.

At present, various fabrics of this type are used for the aforementioned diverse purposes, although they all have various drawbacks. Thus, for example, the oilcloths used as tablecloths have a considerable thickness and weight and although they are easy to wash, their plastic surface is not pleasant to touch. A plastics material is normally used beneath a sheet in the case of mattress covers and sofa protectors. This has various drawbacks, since the plastics material is not breathable, two elements rather than a single element have to be positioned and, furthermore, the sheet slides on the plastic surface and this is unpleasant for the user.

With regard to garments intended to make contact with the user's skin in this type of application, investigations carried out by the inventor demonstrate that, in general, users, in particular users of undergarments, demand that these garments are not externally distinguishable from other garments which are soft to touch, durable and easy to wash, in other words can be washed with neutral detergents that are not aggressive toward the fabrics, have higher breathability than normal undergarments, are comfortable to wear and obviously are waterproof so as to prevent secreted liquids from passing through and staining the external clothes.

At present, however, there are no commercially available undergarments which fulfil all the aforementioned requirements, so users are obliged to take up options which only partially solve the problem.

ES 1036818 and ES 1039554, both from the same inventor, disclose undergarments for this type of application, but merely claim undergarments which have a region of contact with the urethra. Said garments have a fabric composed of three layers joined by heat-soldering, stitched or glued, of which the internal layer in contact with the skin is made of cotton or the like, the preferably breathable waterproof intermediate layer is made of polyurethane or the fabric covered by the registered trade mark Gore Tex and the external layer is also made of cotton or the like. ES 1036818 mentions the possibility of eliminating the internal cotton layer in contact with the skin, while ES 1039554 discloses the possibility of eliminating either of the two textile layers.

Said pants do not meet the aforementioned requirements. The fabrics consisting of three layers joined by the seams are found by users to be bulky and uncomfortable. In addition, the third, thermally joined external layer impairs the breathability of the garment and considerably complicates the production thereof. On the other hand, elimination of one of the three layers leaves a waterproof layer in contact with the skin and this is uncomfortable for the user if body secretions are produced, since the liquids remain in direct contact with the skin, trapped between the skin and the waterproof layer.

On the other hand, although it is normal to select a cotton fabric which is basically composed of cellulose fibres and is known to be comfortable owing to its high hygroscopicity, it cannot be satisfactorily used in combination with a waterproof layer in cases where it will be in contact with the skin, on account of various factors. Firstly, the characteristics of cotton (non-thermoplastic cellulosic fibre which creases easily and shrinks after washing) make it difficult to join it intimately to a layer of waterproof synthetic material. Secondly, a conventional cotton fabric under a layer of limited breathability (which is the case with all known waterproof fabrics), owing to its high degree of water retention, keeps the stain in contact with the skin for a prolonged period and therefore causes a sensation of discomfort for the user. Finally, the stains on the cotton are not easy to wash, necessitating the use of bleach, which rapidly degrades the cotton. With regard to the waterproof layer, the polyurethane mentioned as an example of a waterproof but breathable layer is itself a waterproof but non-breathable material. If this problem was solved by using a commercially available polyurethane membrane having a breathable structure, the moisture originating from the escape of liquid originating from the human body would obstruct the pores of the polyurethane membrane, cancelling its breathability on production of a liquid secretion and causing additional discomfort for the user.

To solve this problem, almost all commercially available waterproof breathable fabrics, including those sold under the trade mark Gore Tex, usually have additionally a hydrophobic surface or layer which, in normal applications, is disposed on the external layer of the garment. However, the cited documents do not disclose the position in which said surface is to be placed.

On the other hand, there is no explicit indication of the requirements of waterproofness and breathability that the used fabric has to fulfil, even though this is important since waterproofness and breathability are opposing concepts and an increase in waterproofness is usually accompanied by a reduction in breathability and *vice versa*. In this respect, it is mentioned in ES 1039554 that this layer must allow adequate breathability from the human body, from which it can be deduced that the level of breathability must be suitable for the normal perspiration of the human body, which is not sufficient, as will be demonstrated hereinafter.

On the other hand, these garments produced in accordance with the instructions in the aforementioned documents lose their waterproofness at the sewn seams required, for example, for obtaining three-dimensional shapes.

ES 1054816, belonging to the inventor of the present patent, discloses a woman's undergarment comprising a fabric with at least two layers, the internal layer being a fabric which is soft to touch and of which the texture is such that it dilutes the stains so that it distributes them over the layer, the layer being easy to wash and having anti-allergic properties. The external layer is made of a material which is breathable and waterproof, at least with regard to the discharge of body secretions. Although a person skilled in the art may obtain a garment which formally meets all the above-described requirements, the drawback arises, as demonstrated by tests carried out by the inventor of this application, that the market demands a higher degree of fulfilment of said objects than is achieved by said fabrics.

The fabric disclosed in said ES 1054816 is considered as the prior art closest to the present invention, and constitutes the preamble of claim 1.

It is an object of the present invention to disclose a fabric of the aforementioned type of which the characteristics have been determined by the inventor after numerous investigations and tests, the fabric surprisingly having characteristics which are far superior to those of other fabrics of its type.

A further object of the present invention is to disclose a fabric which may be used in any type of application requiring a fabric which prevents the passage of liquids through it and which has additional advantages enabling it to overcome the aforementioned drawbacks. For example, mattress covers, sofa protectors, articles of clothing intended to make contact with the skin in the case of conditions whose symptoms include liquid secretions, etc.

A further object of the present invention is to disclose a process for producing said material.

A further object of the present invention is to disclose garments which contain said fabrics.

A further object of the present invention is to disclose undergarments which have seams in the aforementioned fabric which are also waterproof.

More specifically, the present invention consists of a fabric of the type which consists of at least two layers joined together by one face, namely a first layer or internal layer of a fabric which is soft to touch and has a texture which dilutes stains over the layer, is easy to wash and has anti-allergic properties, and a second layer or external layer which comprises a waterproof breathable material, characterised in that the internal layer is composed of a polyester-based fabric which rapidly absorbs the moisture in liquid form present on the free face which is not joined to the external layer and conveys it to the other face of the layer where it dilutes it over it, the free face of the layer having a water retention value of less than 10 %, and in that the external layer is waterproof at least to a pressure equivalent to 5 m H₂O and has breathability of at least 8,000 g/m² every 24 hours measured under conditions described hereinafter in the accompanying detailed description and in that the fabric formed by the two layers has a weight of less than 210 g/m², and preferably less than 180 g/m².

In cases where the waterproof breathable layer requires a hydrophobic surface to prevent the layer from reducing its properties of breathability in the event of becoming wet, the layer will be placed in contact with the internal layer.

In a preferred embodiment, the waterproof breathable layer will have breathability of at least 10,000 g/m² every 24 hours and preferably of more than 15,000 g/m² every 24 hours and even more preferably of more than 21,000 g/m².

In a further preferred embodiment, the external layer will be waterproof to a pressure of at least 8 m H₂O. More preferably, it will be waterproof to a water pressure of at least 10 m H₂O and even more preferably of at least 20 m H₂O.

In a further preferred embodiment, the fabric of the internal layer will have a capillary structure for conveying water to the face of the layer in contact with the external layer.

In a further preferred embodiment, the waterproof breathable fabric will comprise a hydrophobic surface at the interface with said internal layer and a microporous polyurethane coating.

The superior performance of the fabric according to the present invention is due to the fact that said fabric conveys the stain produced by the body secretion to the surface of contact with the waterproof but breathable layer where it is also diluted over the surface. The flow of heat originating from the human body evaporates the moisture present in said surface, allowing the passage thereof by transpiration through the breathable surface. It can be inferred from this that the breathable surface must be capable not only of allowing the passage of transpiration from the human body but also of expelling the moisture of the liquid or semi-liquid secretions by transpiration. Taking this fact into account, the present invention provides, in a novel manner, that the breathability of the waterproof layer must be maximised, while maintaining minimum waterproofness which is adequate for use of the garment. On the other hand, according to the results of the tests and experiments carried out by the inventor, in order for the garment to be comfortable, it must not have a great thickness since an additional novel requirement is that the combination of the fabrics of the layers should be selected in such a way that the fabric does not exceed the maximum indicated weight threshold. The positioning of the hydrophobic surface at the interface with the internal layer is also particularly novel.

The present invention also consists of a production process starting with fabrics of a known type, which comprises the steps of applying a solvent-free non-toxic polyurethane resin at high temperature between the internal and external layers and supplying the fabrics to a laminator which ensures contact therebetween. Preferably, the resin will be heated to a temperature of between 60°C and 100°C and more preferably of approximately 80°C, and the contact between fabrics in the laminator will be made at a velocity of between 0.05 and 0.0625 metres per second. Also preferably, the hydrophobic face of the external layer will make contact with the face intended to evaporate the moisture from the internal layer.

The invention also consists of a textile article which completely or partially comprises a fabric according to the present invention, in which the internal layer of said fabric will preferably be intended to make contact with the user's skin.

In a preferred embodiment, the seams present in the region to be rendered waterproof will be thermally sealed with thermoplastic material to ensure the liquid-tightness thereof.

Some drawings of a preferred embodiment of the present invention are attached as nonlimiting examples, to assist understanding thereof.
Fig. 1 is a schematic view of the fabric according to the present invention showing the face intended to make contact with the skin.
Fig. 2 is a cross-section of a fabric according to the present invention.
Fig. 3 is a schematic view of the fabric according to the present invention showing the face remote from that shown in Fig. 1.
Fig. 4 is a schematic cross-section of a detail of an embodiment of the fabric according to the present invention showing the way in which it reacts to body secretions.
Fig. 5 is a diagram of the process for obtaining the fabric according to the present invention.
Fig. 6 shows a pair of pants containing the fabric according to the present invention, only half of which is shown.
Fig. 7 is a section in the plane VII-VII in Fig. 6 with an enlarged detail.
Fig. 8 shows the internal face of a brassiere produced with the fabric according to the present invention, only one half of which is shown.
Fig. 9 is a section in the plane IX-IX in Fig. 8, with two enlarged details.
Fig. 10 shows an example of waterproof seam produced in the brassiere shown in Fig. 8, viewed from the side of contact with the skin.
Fig. 11 is a cross-section of said seam in the plane XI-XI in Fig. 8.
Fig. 12 corresponds to said waterproof seam, from the side remote from that shown in Fig. 10.

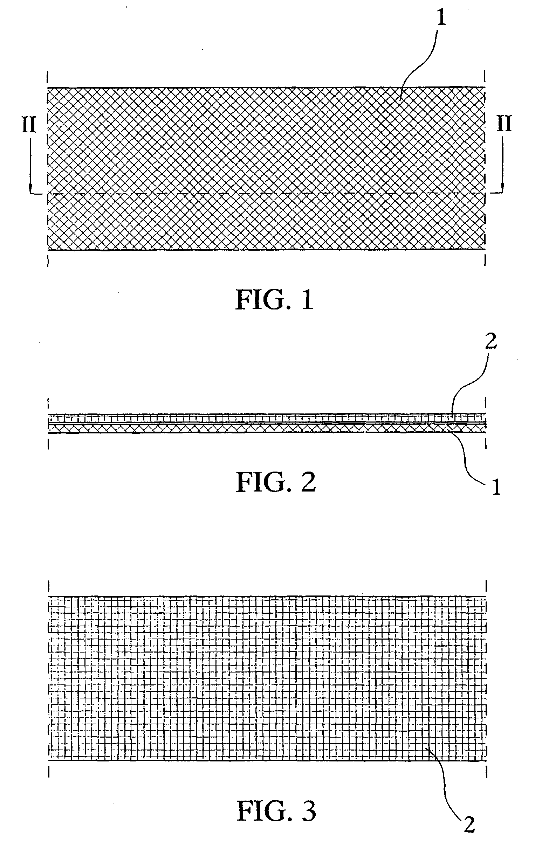

As shown in Fig. 1 to 3, the fabric according to the present invention is composed of two main layers 1 and 2, which may correspond to two types of commercially available fabric and have been joined by heat lamination.

According to the foregoing, the functions to be fulfilled by the external layer are as follows:
- It must prevent the passage of liquid originating from the user's secretions, in other words must prevent the passage of liquid originating from the internal layer.
- It must not lose its properties when washed.
- It must allow the passage of water vapour originating from the region in contact with the user. The present invention provides that the breathability is the maximum possible for drying stains created by body secretions on the internal layer by evaporation.

The functions to be fulfilled by the internal layer in contact with the skin are as follows:
- It must feel pleasant to the user's skin.
- The stains which it has as a consequence of the secretions must readily disappear when neutral detergents are used.
- It must be breathable to allow the water vapour from bodily perspiration to reach the waterproof breathable layer.
- The present invention provides that the fabric rapidly absorbs liquid or semiliquid secretions so as to keep the user dry.
- It must disperse stains over the layer, especially at the surface joined to the external layer.
- The present invention also provides that the layer does not retain liquids originating from body secretions in the region of contact with the skin but rapidly removes said moisture from said region of contact.
- Since these are fabrics which are in contact with bodily mucus, they must preferably have anti-allergic and/or anti-bacterial properties and/or treatments of a known type.

In addition, the combination must have the following properties.
- It must be formed as a single entity.
- According to the present invention, it must be light so that it has a thickness which is comfortable for the user.
- The present invention also provides that the assembly is structurally stable after washing. In other words, neither of the two layers must shrink to a greater extent than the other.

According to the present invention, the internal layer 1 is produced from a polyester-based material.

Polyester has the advantage of being a material with high dimensional stability. This represents an advantage over other materials, such as cotton, which tend to shrink. This is significant because, if the material of the internal layer, but not the fabrics of the external layer, were to shrink, the assembly would bend and become deformed. In addition, polyester has a very soft feel which is suitable for this application and it is resistant both to dirt and to the majority of organic solvents, and this makes it a fibre which is resistant to washing. Traditionally, its poor hygroscopicity made it an uncomfortable fibre. However, there are numerous very well known techniques (for example inclusion of cellulosic fibres, addition of silica, special structures, etc.) for improving the hygroscopicity of polyester and/or the comfort of the fabric without sacrificing other properties. In addition, there are known treatments which impart anti-allergic, deodorant and bactericidal properties to the fabrics thereof.

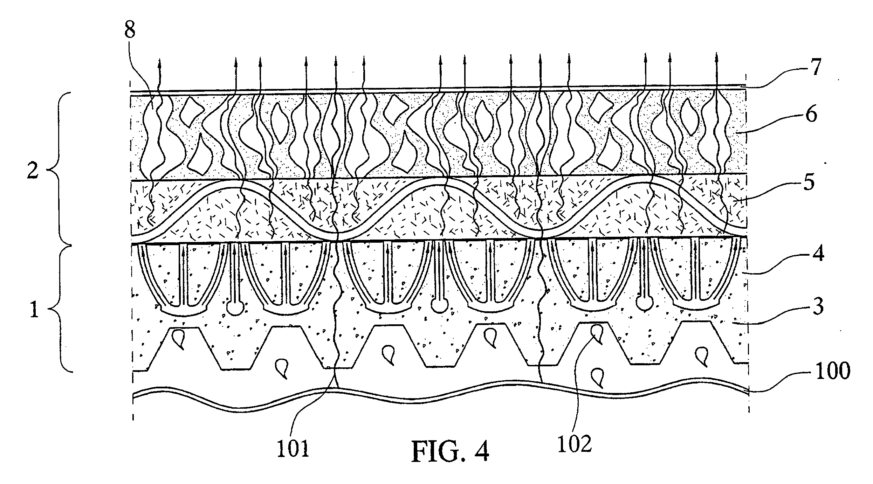

As shown in Fig. 4, which is characteristic of the present invention, the internal layer 1 has a structure which enables it to rapidly absorb liquids on the side which is intended to make contact with the skin, conveying said liquid to the side in contact with the breathable waterproof layer and dispersing said stain over the layer.

Surprisingly, the results achieved with this type of fabric in tests carried out by the present inventors are much better than the results achieved with other materials which are soft to touch and disperse the stain over the layer. Known fabrics of this type, conventionally used for sports applications, include three types of system for achieving said effect: by means of the structure of the fabric, by means of the structure of the yam (which is the system used by the majority of sports fabrics) and by means of a chemical reaction. Although the three systems may suffice, tests carried out by the inventor show that the best results are achieved with the first-mentioned type. Fabrics of this type are conventionally composed of two sub-layers 3 and 4, as shown in the example illustrated schematically in Fig. 4. The sub-layer 3 in contact with the skin has the main function of absorbing liquid and conventionally has a slightly hairy surface of which the surface area decreases in the region next to the skin. The other sub-layer 4 has a structure which forms open capillaries, the function of which is to convey the liquid by capillary action to the surface further removed the skin 100, while dispersing it to facilitate the evaporation thereof.

Although it is necessary, for comfort, that the fabric has a known minimum hygroscopicity, this must not exceed a specific value to prevent liquid retention in the contact region from causing discomfort to the user when secretion occurs. The present invention provides that the water retention of the fabric of the internal layer on the side in contact with the skin is less than 10 %. Higher water retention values mean that the surface of the fabric in contact with the skin gives an unpleasant sensation of moisture. This low water-retention value of the fabric is combined with a high degree of comfort for the user owing to the conveyance of water to the side of the layer which does not make contact with the skin, and the dilution thereof in the direction of the layer.

With regard to the breathable waterproof layer, there are various commercially available technologies.

Virtually all known technologies necessitate the presence of a surface with a hydrophobic treatment 5 to prevent the liquid from accumulating in the waterproof layer 2 and cancelling the breathability thereof. Said fabrics are intended to allow the breathability from the interior to the exterior of the garment, which is why said surface or hydrophobic layer is located at the exterior.

In a novel manner and characteristically of the present invention, said hydrophobic layer 5 is located toward the interior on the surface in contact with the internal layer 1, to prevent the water conveyed through the fabric from the internal layer to the waterproof layer from impeding the breathability thereof.

Fig. 4 shows an example of breathable waterproof layer of a known type composed of a first sub-layer 5 of a nylon fabric which has been given a hydrophobic treatment and a further sub-layer 6 in the form of a microporous sheet of polyurethane and finally an optional non-porous polyurethane membrane 7, which is also breathable.

Fig. 4 also shows the operation of the fabric according to the present invention shown in Fig. 1 to 3.

The water vapour 101 originating from the transpiration of the human body passes through the first layer 1 and through the hydrophobic layer 5, reaching the waterproof membrane 6 where it passes through the micropores 8.

On the other hand, a droplet 102 of secreted liquid, for example a droplet of urine, which reaches the surface of the fabric 1 in contact with the skin 100 is initially rapidly absorbed by the sub-layer 3 and placed in contact with the layer 4, the liquid being removed from contact with the skin and the skin is thus kept dry. The layer 4 conveys the liquid by capillary action to the interface with the breathable waterproof layer and disperses it in the direction of the fabric, thus assisting the evaporation thereof. When the liquid reaches the hydrophobic layer 5, it is repelled so that the droplets of liquid do not wet the micropores 8. On the other hand, the dispersed liquid retained between the layers 4 and 5 is evaporated by the flow of heat from the human body and said vapour escapes to the exterior through the micropores 8. The drying of the stain through the breathable waterproof layer 2 is assisted in this way. On the other hand, the dilution of the stain assists the subsequent washing thereof. The user's comfort is enhanced if the fabric in contact with the skin does not absorb water, and this is achieved by means of the layer 3, which has water retention of less than 10 %.

The conveyance and dilution of the liquid of the stain toward the breathable waterproof layer 2 for the drying thereof through it, led the inventor to the conclusion that said layer 2 should maximise its permeability.

It is conventionally considered that a fabric is breathable if it is capable of transmitting 1,000 g/m²/24 h, in other words if it is capable of permitting the passage through it of 1,000 g of water in vapour form for each square metre of cloth in a time period of 24 hours.

The tests carried out show that the fabrics for the layer 2 which have a breathability value more than 8,000 g/m²/24 h give good results in applications involving occasional, light secretions. However, said value will preferably be 10,000 g/m²/24 h. For applications where secretions may be frequent and/or considerable, the breathability will preferably be 21,000 g/m²/24 h.

The breathability of a fabric depends on the surrounding environmental conditions. In this specification, the quoted breathability values refer to values obtained in conditions as specified in the standard JIS L 1099, inverted cup method B-1.

With regard to the waterproofness, the fabrics sold under the trade mark Gore Tex and the like, which are based on expanded PTFE (polytetrafluoroethylene) technology, ensure maximum waterproofness of more than 27 m H₂O. This means that the fabric is capable of withstanding a 27 metre high water column on it, without allowing the passage of water. For the current application, such a degree of waterproofness is not required and may be expensive and/or may lead to lesser breathability, or in general to a thicker fabric, which is undesirable.

A fabric having waterproofness of more than 2 m H₂O is conventionally considered to be waterproof. On the other hand, it is considered that a fabric must have waterproofness of 8 m H₂O to ensure that a person sitting on a wet surface will be kept dry. The tests carried out by the inventor show that, for occasional losses of low intensity, waterproof fabrics which ensure waterproofness to 5 m H₂O are sufficient owing to the presence of the internal layer 1. For garments intended for active use or in cases of frequent and/or significant secretions, the waterproofness may increase to at least 10 or 20 m H₂O.

On the other hand, the fabric obtained by joining the layers 1 and 2 must not be thick. This means that it must not be heavy. In particular, its weight must not exceed 210 g/m² and, more preferably, 180 g/m².

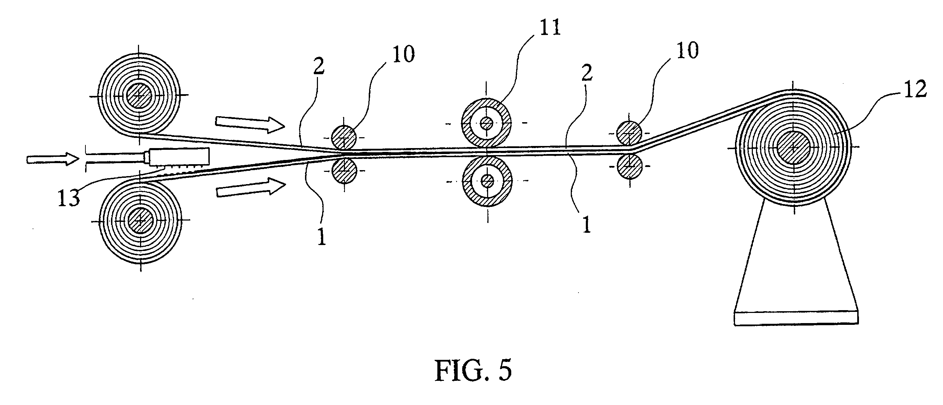

Fig. 5 shows a process for joining two commercially available fabrics corresponding to the above-described layers 1 and 2, by lamination. The two layers 1 and 2 are superimposed using cylinders 10. Before bringing the layers into contact, a polyurethane resin 13 is applied, to join the two layers 1 and 2, at an approximate temperature of 80°C. Owing to the application of the fabric, it is important that the resin 13 does not contain solvents and is not toxic. It is also important to maintain the breathability through the joining of layers, for which purpose it is necessary to use an amount of resin which is minimal but is sufficient to produce an adequate joint between the two layers 1 and 2 in such a way that the resin forms the finest possible membrane between the fabrics 1 and 2 on completion of the joining process and/or that it is not interposed exhaustively therebetween over their entire extension.

The layers 1 and 2 must be placed in such a way that the surfaces 4 and 5 make mutual contact. The two fabrics are subsequently passed through a laminator 11 which ensures contact between the two fabrics at a velocity of between 0.05 and 0.0625 metres per second. As a result, the two layers leave the laminator 11 as a single entity and may then optionally be rolled in the reel 12 in a known manner.

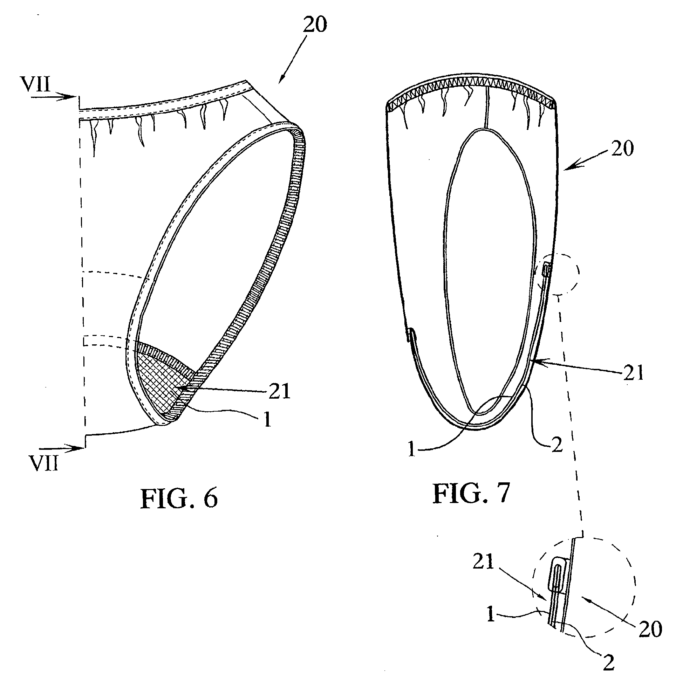

Fig. 6 and 7 show a first example of undergarment which employs the fabric according to the present invention and which consists of women's pants in this case. In the example shown, the main body of the pants 2 is produced in a known shape with known materials. In its lower portion, by the internal face intended to make contact with the skin, there is placed a portion 21 of the fabric according to the present invention, joined to the body of the pants 20 via the periphery thereof in such a way that the layer 1 is in contact with the skin and the layer 2 is located between said layer and the body of the pants 20. These pants are suitable for menstrual periods, and they protect the user from losses through a pad or tampon and meet the aforementioned requirements.

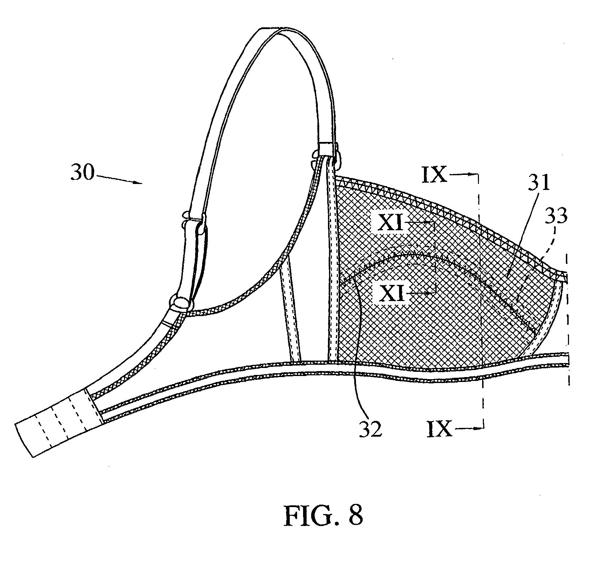

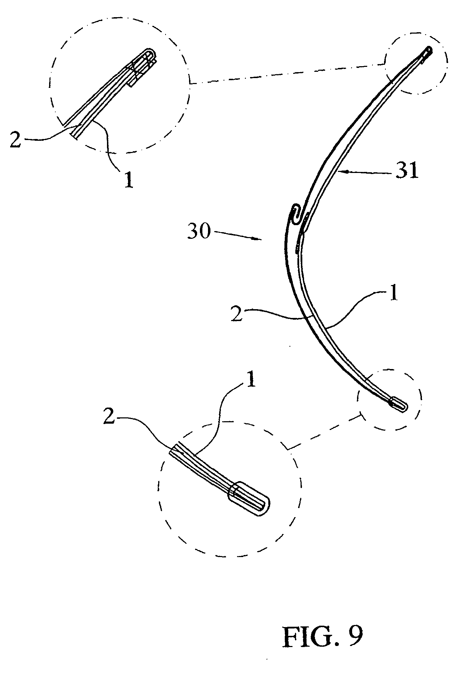

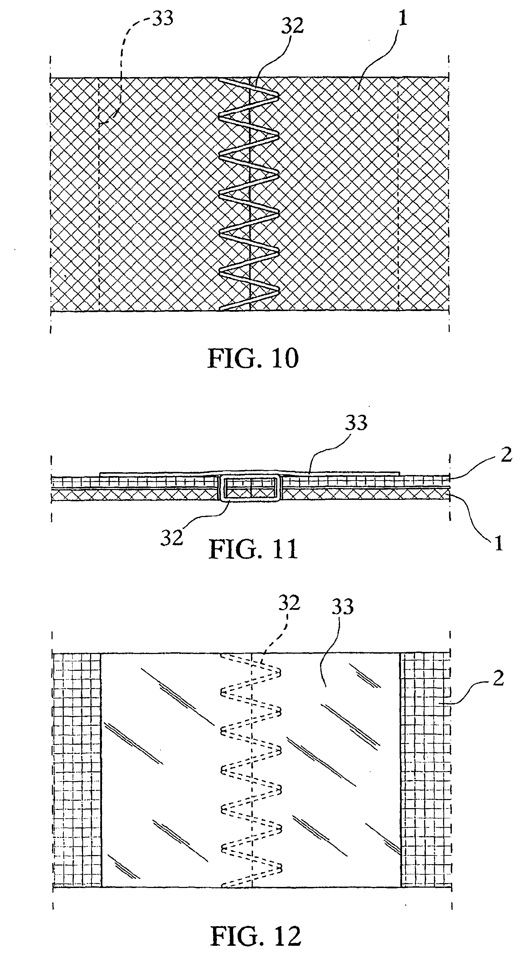

Fig. 8 to 12 show a further example consisting of a brassiere 30 suitable for losses of maternal milk and other mammary secretions. The structure of the brassiere 30 is identical to that of a conventional brassiere. The fabric in the interior of the cups 31 is the fabric according to the present invention, again with the layer 1 in contact with the user's skin. The cup 31 has a three-dimensional shape, necessitating the production of a seam 32. In a manner which is characteristic of the present invention, said seam which consists of a conventional seam, for example an abutting seam sewn with a double thread, has the feature that, through the part of the fabric which is not in contact with the skin, in other words through the layer 2, the seam 32 has been covered with a strip 33 of a thermoplastic material which has been soldered by a known means, for example heat lamination. The seam 32 is thus made waterproof and prevents losses of liquid through it.

The examples in Fig. 6 to 12 are merely illustrative examples of the application of the fabric according to the present invention to undergarments. However, the shape and type of undergarment is arbitrary, the fabric according to the present invention being used in part or the entire garment being produced from this fabric. The fabric according to the present invention may obviously also be applied to other types of element such as mattress covers, tablecloths, sofa protectors, hospital beds and, in general, any type of clothing in which it is useful to employ a fabric having the characteristic described by the present invention. Fabrics which comply with the described specifications and are obtained by a different process from that described here are also deemed to be included within the present invention.

## Claims

1. Fabric, of the type which is composed of two layers joined together by one face, namely a first layer or internal layer of a fabric which is soft to touch and has a staindiluting layer, is easy to wash and has anti-allergic properties, and a second layer or external layer which comprises a waterproof breathable material, **characterised in that** the internal layer is composed of a polyester-based fabric which absorbs the moisture in liquid form present on the free face which is not joined to the external layer and conveys it to the other face of the internal layer where it dilutes it over the surface, the fabric at the free face of the internal layer having a water retention value of less than 10 %, and **in that** the external layer is waterproof at least to a pressure equivalent to 5 m H₂O and has breathability of more than 8,000 g/m² every 24 hours and **in that** the fabric formed by the two layers has a weight of less than 210 g/m².

2. Fabric according to claim 1, **characterised in that** the external layer has a hydrophobic surface in contact with the internal layer.

3. Fabric according to either claim 1 or claim 2, **characterised in that** the fabric has a weight of less than 180 g/m².

4. Fabric according to any one of claims 1 to 3, **characterised in that** the external layer has breathability of more than 10,000 g/m² every 24 hours.

5. Fabric according to any one of claims 1 to 3, **characterised in that** the external layer has breathability of more than 15,000 g/m² every 24 hours.

6. Fabric according to any one of claims 1 to 3, **characterised in that** the external layer has breathability of more than 21,000 g/m² every 24 hours.

7. Fabric according to any one of claims 1 to 6, **characterised in that** the external layer is waterproof to a pressure of at least 8 m H₂O.

8. Fabric according to any one of claims 1 to 6, **characterised in that** the external layer is waterproof to a pressure of at least 10 m H₂O.

9. Fabric according to any one of claims 1 to 6, **characterised in that** the external layer is waterproof to a pressure of at least 20 m H₂O.

10. Fabric according to any one of claims 1 to 9, **characterised in that** the internal layer has a capillary structure for conveying water to the face of the layer in contact with the external layer.

11. Fabric according to claim 10, **characterised in that** the internal layer is composed of two sub-layers, the first having a hairy surface and a surface area which decreases in the region of contact with the skin and having the characteristic of rapid absorption of liquids and the second having an open structure of which the function is to convey the liquid to the surface by capillary action while dispersing it.

12. Fabric according to any one of claims 1 to 11, **characterised in that** the external layer has a hydrophobic surface at the interface with the internal layer and a microporous polyurethane coating.

13. Process for producing a fabric according to any one of claims 1 to 12, starting with fabrics of a known type, **characterised in that** it comprises the steps of applying a solvent-free non-toxic polyurethane resin at high temperature between the internal and external layers and supplying the fabrics to a laminator which ensures contact therebetween.

14. Process for producing a fabric according to claim 13, **characterised in that** the resin is heated to a temperature of between 60 and 100°C.

15. Process for producing a fabric according to claim 14, **characterised in that** the resin is heated to a temperature of 80°C.

16. Process according to any one of claims 13 to 15, **characterised in that** the contact between fabrics in the laminator is made at a velocity of between 16 and 20 seconds per metre.

17. Process according to any one of claims 13 to 16, **characterised in that** the hydrophobic surface of the external face is placed in contact with the face intended to evaporate the moisture from the internal layer.

18. Textile article, **characterised in that** it completely or partially comprises a fabric according to any one of claims 1 to 12.

19. Textile article according to claim 18, **characterised in that** it comprises seams which are covered on the external face of the fabric with a strip of thermoplastic material which seals said seam.

20. Textile article according to either claim 18 or claim 19, **characterised in that** it consists of a garment of which the internal layer is intended to make contact with the user's skin.

21. Textile article according to claim 20, **characterised in that** the garment is an undergarment.

## Patentansprüche

1. GewebederArt, welche aus zwei Schichten besteht, die miteinander durch eineFläche verbunden sind, nämlich einer ersten Schicht oder Innenschicht aus einem Gewebe, das weich anzufassen ist und eine Fleckvermindernde Schicht aufweist, einfach zu waschen ist und anti-allergene Eigenschaftenhat, und einer zweiten Schicht oder Außenschicht, welche ein wasserdichtes, atmungsaktives Material umfasst,
**dadurch gekennzeichnet, dass** die Innenschicht aus einem Polyester-basierten Gewebe besteht, welches die Feuchtigkeit in flüssiger Form, die auf der freien Seite, welche nicht mit der Außenschicht verbunden ist, vorliegt, absorbiert und diese zu der anderen Fläche der Innenschicht befördert, wo es diese über die Oberfläche verdünnt, wobei das Gewebe an der freien Fläche der Innenschicht einen Wasserretentionswert von weniger als 10% aufweist, und dass die Außenschicht mindestens bis zu einem Druckäquivalent von 5 m H₂O wasserdicht istund eine Atmungsaktivität von mehr als 8000 g/m² je 24 Stunden aufweist, und dass das Gewebe, welches aus den beiden Schichten gebildet wird, ein Gewicht von weniger als 210 g/m² aufweist.

2. Gewebe nach Anspruch 1, **dadurch gekennzeichnet, dass** die Außenschicht eine hydrophobe Oberfläche in Kontakt mit der Innenschicht aufweist.

3. Gewebe nach Anspruch 1 oder Anspruch 2, **dadurch gekennzeichnet, dass** das Gewebe ein Gewicht von weniger als 180 g/m² aufweist.

4. Gewebe nach einem der Ansprüche 1 bis 3, **dadurch gekennzeichnet, dass** die Außenschicht eine Atmungsaktivität von mehr als 10.000 g/m²je 24 Stunden aufweist.

5. Gewebe nach einem der Ansprüche 1 bis 3, **dadurch gekennzeichnet, dass** die Außenschicht eine Atmungsaktivität von mehr als 15.000 g/m²je 24 Stunden aufweist.

6. Gewebe nach einem der Ansprüche 1 bis 3, **dadurch gekennzeichnet, dass** die Außenschicht eine Atmungsaktivität von mehr als 21.000 g/m²je 24 Stunden aufweist.

7. Gewebe nach einem der Ansprüche 1 bis 6, **dadurch gekennzeichnet, dass** die Außenschicht bis zu einem Druck von mindestens 8 m H₂O wasserdicht ist.

8. Gewebe nach einem der Ansprüche 1 bis 6, **dadurch gekennzeichnet, dass** die Außenschicht bis zu einem Druck von mindestens 10 m H₂O wasserdicht ist.

9. Gewebe nach einem der Ansprüche 1 bis 6, **dadurch gekennzeichnet, dass** die Außenschicht bis zu einem Druck von mindestens 20 m H₂O wasserdicht ist.

10. Gewebe nach einem der Ansprüche 1 bis 9, **dadurch gekennzeichnet, dass** die Innenschicht eine kapillare Struktur zum Befördern von Wasser zu der Fläche der Schicht, die in Kontakt mit der Außenschicht steht, aufweist.

11. Gewebe nach Anspruch 10, **dadurch gekennzeichnet, dass** die Innenschicht aus zwei Unterschichten besteht, wobei die Erste eine haarige Oberfläche und eine Oberfläche aufweist, die im Bereich desKontakts mit der Haut kleiner wird und die Eigenschaft einer schnellen Absorption von Flüssigkeiten aufweist und wobei die Zweite eine offene Struktur aufweist, deren Funktion es ist, die Flüssigkeit durch Kapillarwirkung an die Oberfläche zu befördern und dabei fein zu verteilen.

12. Gewebe nach einem der Ansprüche 1 bis 11, **dadurch gekennzeichnet, dass** die Außenschicht eine hydrophobe Oberfläche an der Grenzfläche mit der Innenschicht und eine mikroporöse Polyurethan-Beschichtung aufweist.

13. Verfahren zur Herstellung eines Gewebes nach einem der Ansprüche 1 bis 12, welches mit Geweben eines bekannten Typs beginnt, **dadurch gekennzeichnet, dass** es die Schritte des Aufbringens eines lösemittelfreien, nicht-toxischen Polyurethanharzes bei hoher Temperatur zwischen den InnenundAußenschichten und des Zuführens der Gewebe zu einem Laminiergerät umfasst, dasden Kontakt zwischen ihnen sicherstellt.

14. Verfahren zur Herstellung eines Gewebes nach Anspruch 13, **dadurch gekennzeichnet**, dassdas Harz auf eine Temperatur zwischen 60 und 100°C erhitzt wird.

15. Verfahren zur Herstellung eines Gewebes nach Anspruch 14, **dadurch gekennzeichnet**, dassdas Harz auf eine Temperatur von 80°C erhitzt wird.

16. Verfahren nach einem der Ansprüche 13 bis 15, **dadurch gekennzeichnet, dass** der Kontakt zwischen den Geweben im Laminiergerätbei einer Geschwindigkeit zwischen 16 und 20 Sekunden pro Meter hergestellt wird.

17. Verfahren nach einem der Ansprüche 13 bis 16, **dadurch gekennzeichnet, dass** die hydrophobe Oberfläche der Außenfläche in Kontakt mit derjenigen Fläche gesetzt wird, die dazu bestimmt ist, die Feuchtigkeit von der Innenschicht zu verdampfen.

18. Textilartikel, **dadurch gekennzeichnet, dass** es ein Gewebe nach einem der Ansprüche 1 bis 12 umfasstvollständig oder teilweise umfasst.

19. Textilartikelgemäß Anspruch 18, **dadurch gekennzeichnet, dass** er Nähte umfasst, die auf der Außenfläche des Gewebes mit einem Streifen aus thermoplastischem Material bedeckt sind, welcher diese versiegelt.

20. Textilartikelgemäß Anspruch 18 oder Anspruch 19, **dadurch gekennzeichnet, dass** er aus einem Kleidungsstück besteht, dessenlnnenschicht dazu bestimmt ist, den Kontakt mit der Haut des Benutzers herzustellen.

21. Textilartikel gemäß Anspruch 20, **dadurch gekennzeichnet, dass** das Kleidungsstück ein Unterbekleidungsstück ist.

## Revendications

1. Tissu, du type qui est constitué de deux couches liées ensemble par une face, à savoir une première couche ou couche interne d'un tissu qui est souple au toucher et qui a une couche de dilution des taches, qui est facile à laver et qui a des propriété anti-allergéniques, et une seconde couche ou couche externe qui est constituée d'un matériau respirant imperméable à l'eau, **caractérisé en ce que** la couche interne est constituée d'un tissu à base de polyester qui absorbe l'humidité sous forme liquide présente sur la face libre qui n'est pas liée à la couche externe et qui la transporte vers l'autre face de la couche interne où elle la dilue sur la surface, le tissu au niveau de la face libre de la couche interne ayant une valeur de rétention d'eau inférieure à 10 % et **en ce que** la couche externe est imperméable à l'eau à une pression au moins équivalente à 5 m de H₂O et a une capacité de respiration supérieure à 8 000 g/m² toutes les 24 heures et **en ce que** le tissu formé par les deux couches a un poids inférieur à 210 g/m².

2. Tissu selon la revendication 1, **caractérisé en ce que** la couche externe a une surface hydrophobe en contact avec la couche interne.

3. Tissu selon la revendication 1 ou la revendication 2, **caractérisé en ce que** le tissu a un poids inférieur à 180 g/m².

4. Tissu selon l'une quelconque des revendications 1 à 3, **caractérisé en ce que** la couche externe a une capacité de respiration supérieure à 10 000 g/m² toutes les 24 heures.

5. Tissu selon l'une quelconque des revendications 1 à 3, **caractérisé en ce que** la couche externe a une capacité de respiration supérieure à 15 000 g/m² toutes les 24 heures.

6. Tissu selon l'une quelconque des revendications 1 à 3, **caractérisé en ce que** la couche externe a une capacité de respiration supérieure à 21 000 g/m² toutes les 24 heures.

7. Tissu selon l'une quelconque des revendications 1 à 6, **caractérisé en ce que** la couche externe est imperméable à l'eau à une pression d'au moins 8 m de H₂O.

8. Tissu selon l'une quelconque des revendications 1 à 6, **caractérisé en ce que** la couche externe est imperméable à l'eau à une pression d'au moins 10 m de H₂O.

9. Tissu selon l'une quelconque des revendications 1 à 6, **caractérisé en ce que** la couche externe est imperméable à l'eau à une pression d'au moins 20 m de H₂O.

10. Tissu selon l'une quelconque des revendications 1 à 9, **caractérisé en ce que** la couche interne a une structure capillaire pour transporter l'eau vers la face de la couche en contact avec la couche externe.

11. Tissu selon la revendication 10, **caractérisé en ce que** la couche interne est constituée de deux sous-couches, la première ayant une surface duveteuse et une surface spécifique qui diminue dans la région de contact avec la peau et ayant la caractéristique d'une absorption rapide des liquides et la seconde ayant une structure ouverte dont la fonction est de transporter le liquide vers la surface par une action capillaire tout en le répartissant.

12. Tissu selon l'une quelconque des revendications 1 à 11, **caractérisé en ce que** la couche externe a une surface hydrophobe à l'interface avec la couche interne et un revêtement en polyuréthane microporeux.

13. Procédé de production d'un tissu selon l'une quelconque des revendications 1 à 12, à partir de tissus d'un type connu, **caractérisé en ce qu'**il comprend les étapes d'application d'une résine de polyuréthane dépourvue de solvant non toxique à une température élevée entre les couches interne et externe et d'alimentation des tissu dans un laminoir qui assure le contact entre celles-ci.

14. Procédé de production d'un tissu selon la revendication 13, **caractérisé en ce que** la résine est chauffée à une température comprise entre 60 et 100°C.

15. Procédé de production d'un tissu selon la revendication 14, **caractérisé en ce que** la résine est chauffée à une température de 80°C.

16. Procédé selon l'une quelconque des revendications 13 à 15, **caractérisé en ce que** le contact entre les tissus dans le laminoir est réalisé à une vitesse comprise entre 16 et 20 secondes par mètre.

17. Procédé selon l'une quelconque des revendications 13 à 16, **caractérisé en ce que** la surface hydrophobe de la face externe est mise en contact avec la face destinée à évaporer l'humidité hors de la couche interne.

18. Article textile, **caractérisé en ce qu'**il comprend complètement ou partiellement un tissu selon l'une quelconque des revendications 1 à 12.

19. Article textile selon la revendication 18, **caractérisé en ce qu'**il comprend des coutures qui sont recouvertes sur la face externe du tissu par une bande de matériau thermoplastique qui scelle ladite couture.

20. Article textile selon la revendication 18 ou la revendication 19, **caractérisé en ce qu'**il constitue un vêtement dont la couche interne est destinée à entrer en contact avec la peau de l'utilisateur.

21. Article textile selon la revendication 20, **caractérisé en ce que** le vêtement est un sous-vêtement.
